Europäisches Patentamt

European Patent Office

Office européen des brevets

(11) Veröffentlichungsnummer: **0 428 060 A1**

# (12) EUROPÄISCHE PATENTANMELDUNG

(21) Anmeldenummer: 90121349.6

(22) Anmeldetag: 08.11.90

(51) Int. Cl.5: **C12N 9/22**, C12N 1/20,
//(C12N1/20,C12R1:41)

Der Anmelder hat nachträglich ein Sequenzprotokoll eingereicht und erklärt, dass dieses keine neuen Angaben enthält.

(30) Priorität: 16.11.89 DE 3938145
07.03.90 DE 4007043

(43) Veröffentlichungstag der Anmeldung:
22.05.91 Patentblatt 91/21

(84) Benannte Vertragsstaaten:
AT BE CH DE DK ES FR GB GR IT LI LU NL SE

(71) Anmelder: BOEHRINGER MANNHEIM GMBH
Sandhofer Strasse 116
W-6800 Mannheim 31(DE)

(72) Erfinder: Kaluza, Klaus, Dr.rer.nat.
Hochfeldanger 3
W-8173 Bad Heilbrunn(DE)
Erfinder: Jarsch, Michael, Dr.rer.nat.
Unterkarpfsee 11
W-8173 Bad Heilbrunn(DE)
Erfinder: Schmitz-Agheguian, Gudrun,
Dr.rer.nat.
Wettersteinstrasse 3
W-8139 Bernried(DE)
Erfinder: Kessler, Christoph, Dr.rer.nat.
Schlossbergweg 11
W-8021 Dorfen(DE)

(54) TYP II-Restriktionsendonuklease R1eAI.

(57) Die neue TypII-Restriktionsendonuklease RleAI besitzt folgende Erkennungssequenz (SEQ ID NO 1), spaltet bevorzugt an der durch die Markierung definierten Spaltstelle:

```
5'-CCC ACA NNNNNNNNN NNN|N-3'
3'-GGG TGT NNNNNNNNN|NNN N-5'
```

(N:A,G,C oder T)

Sie ist vorzugsweise aus Mikroorganismen der Gattung Rhizobium erhältlich.

## TYP II-RESTRIKTIONSENDONUKLEASE RLEAI

Die Erfindung betrifft die neue Typ II-Restriktionsendonuklease RleAI, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiester-brücken in der Zielsequenz hydrolysiert und zwar in jedem Polynukleotidstrang eine. Typ II-Restriktions-endonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht immer noch Bedarf an der Schaffung weiterer Typ II-Restriktionsendonukleasen, die für DNA-Sequenzen spezifisch sind und die bisher von keiner der bekannten Restriktionsendonukleasen erkannt werden. Der Erfindung liegt daher die Aufgabe zu Grunde, eine neue Restriktionsendonuklease zur Verfügung zu stellen, welche eine bisher von keinem derartigen Enzym erkannte Sequenz spezifisch zu erkennen und zu spalten vermag.

Gelöst wird diese Aufgabe erfindungsgemäß durch eine Typ-II-Restriktionsendonuklease mit der Erkennungs-sequenz (SEQ ID NO 1) und der durch die Markierung definierten Spaltstelle

$$5'-\text{CCC ACA NNNNNNNNN NNN} | \text{N}-3'$$
$$3'-\text{GGG TGT NNNNNNNNN} | \text{NNN N}-5'$$

$$(N:A,G,C \text{ oder } T)$$

Die erfindungsgemäße neue Restriktionsendonuklease, die im folgenden als RleAI bezeichnet wird, hat ein Temperaturoptimum bei 30° C. Das Enzym besitzt eine gute Aktivität zwischen pH 7,5 und pH 8,0 bei 10 mmol/l Tris/HCl, 10 mmol/l MgCl$_2$ und 1 mmol/l DTE (Dithioerythritol).

Das pH-Optimum liegt bei pH 7,5. Ein Enzym, das isoschizomer zu RleAI ist, ist nicht bekannt.

Die Erkennungssequenz läßt sich durch vollständigen Verdau der DNAs der Viren SV40 und Adeno 2, der Phagen Lambda, phiX174 und dem Phagenderivaten M13mp8 bestätigen. Diese DNA-Moleküle werden mit RleAI behandelt.

Tabelle 1 zeigt einen Vergleich der experimentell beobachteten Schnittstellenspezifität mit einer computerermittelten Schnittstellenspezifität für ein Enzym, welches die Sequenz CCCACA(N)$_{12/9}$ (SEQ ID NO 1) erkennt.

Tabelle I

| DNA | Anzahl der experimentell bestimmten Schnittstellen | Anzahl der durch Computeranalyse bestimmte Schnittstellen | Experimentell bestimmte Fragmentlängen (Basenpaare) | Durch Computeranalyse bestimmte Fragmentlängen (Basenpaare) | Spaltpositionen ermittelt durch Computeranalyse (bei Basenpaar) |
|---|---|---|---|---|---|
| SV40 | 5 | 5 | 2250 | 2296 | 1825 |
| | | | 1300 | 1272 | 2298 |
| | | | 790 | 793 | 2707 |
| | | | 470 | 473 | 3500 |
| | | | 400 | 409 | 4772 |
| M13mp8 | 1 | 1 | 7200 | 7229 | 3009 |
| phiX174 | 1 | 1 | 5400 | 5386 | 576 |

EP 0 428 060 A1

Die Spaltposition innerhalb der Erkennungssequenz des Enzyms läßt sich an einem M13-Derivat, das im Abstand von ca. 30 -200 Basen zur Bindungsstelle des universalen Sequenzierprimers (Messing, J. et al. (1981) Nucl. Acids Res. 9, 309 - 321) diese Erkennungssequenz trägt, bestimmen. An einzelsträngiger DNA des M13-Derivates werden zunächst mit dem universalen Sequenzierprimer Sequenzreaktionen nach der Didesoxy-Kettenabbruchmethode durchgeführt (Sanger, F., et al. (1977) Proc. Natl. Acad. Sci. USA 74, 560 - 564, Messing, J. et al. (1981) Nucl. Acids Res. 9, 309 -321).

Parallel dazu wird der Sequenzierprimer mit T4-Polynukleotid-Kinase und [γ-$^{32}$P]ATP am 5'-Ende radioaktiv markiert. Nach Anhybridisieren dieses 5'-endmarkierten Sequenzierprimers an die einzelsträngige M13-DNA wird in einer Auffüllreaktion mit DNA-Poly-merase I (Klenow-Enzym) und einer Desoxynukleotidtri-phosphatmischung aus dATP, dCTP, dGTP und dTTP eine partiell doppelsträngige DNA hergestellt. Diese DNA, deren neusynthetisierter Strang am 5'-Ende radioaktiv markiert ist, wird mit der Restriktionsendonuklease RleAI gespalten. Die Hälfte des Spaltansatzes wird zusätzlich noch mit T4 DNA Polymerase in Gegenwart einer Mischung aller vier Desoxynukleotidtriphosphate behandelt, um glatte DNA-Enden zu erhalten.

Die Analyse der Reaktionsprodukte erfolgt durch Elektrophorese auf Sequenziergelen (8 mol/l Harnstoff, 5% Polyacrylamid) und anschließender Autoradiographie. Die Interpretation der Ergebnisse wird nach Brown, N.L. und Smith, M. (Methods in Enzymology 65 (1980) 391 - 401) durchgeführt. Durch Vergleich der Laufstrecken der radioaktiv markierten Fragmente mit der Sequenzleiter wird die Lage der Spaltstelle bestimmt. Die zusätzlich mit T4 DNA Polymerase behandelten Proben zeigen im Vergleich zu der lediglich mit RleAI gespaltenen Probe um drei Nukleotide verllängerte Laufstrecken der Banden. Damit ist gezeigt, daß RleAI 3' überhängende DNA-Enden erzeugt. Die Spaltung von RleAI erfolgt innerhalb der Erkennungssequenz (SEQ ID NO 1) daher mit folgender Spezifität:

$$5'\text{-CCC ACA NNNNNNNN NNN} \big| \text{N-3'}$$
$$3'\text{-GGG TGT NNNNNNNN} \big| \text{NNN N-5'}$$

Die experimentell bestimmte Anzahl der Schnittstellen ist identisch mit der Anzahl der Schnittstellen, die durch Computeranalyse mit den verschiedenen DNAs für die Sequenz CCCACA (N)$_{12/9}$ erhalten wurde (Tabelle I). Zusätzlich wurden diese Daten noch mit den Tabellen aus Gene 10 (1980, 357 -370, verglichen. Ein doppelter Verdau von SV40 DNA mit BamH1 und RleAI bestätigt, das die Spezifität CCCACA(N)$_{12/9}$ ist.

Vorzugsweise wird RleAI gewonnen, indem man Mikro-organismen der Gattung Rhizobium züchtet und das Enzym aus den Zellen gewinnt. Vorzugsweise wird Rhizobium leguminosarum DSM 5629 verwendet.

Der Mikroorganismus Rhizobium leguminosarum ist bei der Deutschen Sammlung von Mikroorganismen (DSM), Mascheroder Weg 1 b, 3300 Braunschweig, BRD, hinterlegt und trägt die Hinterlegungsnummer DSM 5629.

Die für die Gewinnung des Enzyms verwendeten Mikroorganismen wachsen aerob in PSY-Medium:
0,3 g/l $KH_2PO_4$
0,3 g/l $Na_2HPO_4$
0,1 g/l $MgSO_4.7H_2O$
0,07 g/l $CaCl_2.2H_2O$
3 g/l Pepton
1 g/l Hefeextrakt
2 ml/l Spurenelemente 500 x (*)
1 ml/l Vitamine 1000 x (**)
(*) - Spurenelemente 500 x:
5 g/l $H_3BO_3$
500 mg/l $ZnSO_4.7H_2O$
250 mg/l $CuSO_4.5H_2O$
250 mg/l $MnCl_2.4H_2O$
50 mg/l $NaMoO_4.2H_2O$
835 mg/l $FeCl_3.6H_2O$
(**) - Vitamine 1000 x:
1 g/l Thiamin
1 g/l Biotin

1 g/l Pantothenat

Die optimalen Wachstumsbedingungen sind bei 28° C, pH 7,0. Die Verdoppelungszeit beträgt etwa 2,5 Stunden.

Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen über eine French-Presse aufgeschlossen. Die weitere Reinigung des Überstands wird vorzugsweise über Affinitätschromatographie, und Ionentauscher-chromatographie durchgeführt. Als Material für die Affinitätschromatographie geeignet ist beispielsweise Heparin-Sepharose CL-6B (Pharmacia).

Als Anionentauscher geeignet ist das unter dem Namen DEAE

# Sephadex¢

(Pharmacia) erhältliche Produkt. Aber auch andere Chromatographiematerialien, die dem Fachmann bekannt sind, sind geeignet.

Die folgenden Beispiele erläutern die Erfindung weiter.

## Beispiel 1

Rhizobium leguminosarum DSM 5629 wird bei 28°C 32 Stun-den gezüchtet und in der stationären Phase geerntet. Als Kulturmedium wird PSY-Medium verwendet.

Die Zellpaste (30 g Naßgewicht) wird in 2 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch einmalige Passage einer French-Presse bei 23.000 lb/inch$^2$ aufgeschlossen und der Niederschlag abgetrennt. Der Überstand wird anschließend an einer mit Puffer B (40 mmol/Tris-HCl pH 8,5, 0,1 mmol/l EDTA 7 mmol/l 2-Mercaptoethanol, 10% (v/v) Glyzerin) equilibrierten Heparin-Sepharose-Säule fraktioniert. Zur Elution wird ein Gradient von 0 - 1 mol/l NaCl verwendet. RleAl wird in den Fraktionen zwischen 0,4 und 0,6 mol/l NaCl gefunden. Die aktiven Fraktionen werden auf einer Sephadex G 25-Säule entsalzt, anschließend werden sie auf einer Q-Sepharose-Säule, welche mit Puffer B equilibriert wurde, aufgegeben. Zur Elution wird ein Gradient von 0 - 0,5 mol/l NaCl in Puffer B (pH 7,0) verwendet. RleAl wird in den Fraktionen zwischen 0,1 und 0,2 mol/l NaCl gefunden.

Die aktiven Fraktionen werden zusammengegeben und gegen Lagerpuffer (20 mmol/l Tris-HCl, pH 8,0, 10 mmol/l 2-Mercaptoethanol, 100 mmol/l NaCl, 0,1 mmol/l EDTA und 50% (v/v) Glycerin) dialysiert.

## Beispiel 2

Bestimmung der Aktivität

Definition der Enzymeinheiten:

1 U RleAl spaltet 1 μg Lambda-DNA innerhalb 1 Stunde bei 30° C in 25 μl Endvolumen.

Zu einer Mischung von 2,5 μl Inkubationspuffer (100 mmol/l Tris-HCl, pH 7,5/37°C, 100 mmol/l Magnesium-chlorid und 10 mmol/l DTE) werden 17,5 μl Wasser und 5 μl Lambda DNA (optische Diohte: 4 OD/ml) sowie 1 μl RleAl-Lösung (1 U/μl) zugegeben. Die Lösung wird eine Stunde bei 30° C inkubiert, auf Eis gekühlt und mit 5 μl eines Abstopp-Reagenses, bestehend aus 7 mmol/l Harnstoff, 20 % (w/v) Saccharose, 60 mmol/l EDTA und 0,01 % (w/v) Bromphenolblau versetzt. Anschließend wird eine Trennung durch Elektrophorese in 1 % Agarose-Gelen für 3 - 4 Stunden bei 100 V durchgeführt. Die erhaltenen Banden werden über Vergleich mit einem DNA-Längen-standard identifiziert.

**Ansprüche**

1. Typ II-Restriktionsendonuklease mit der Erkennungssequenz (SEQ ID NO 1) und der durch die Markierung charakterisierten Schnittstelle

$$5'\text{-CCC ACA NNNNNNNN NNN} | \text{N-3'}$$
$$3'\text{-GGG TGT NNNNNNNN} | \text{NNN N-5'}$$

(N:A,G,C oder T)

2. Typ II-Restriktionsendonuklease nach Anspruch 1 dadurch gekennzeichnet, daß sie aus Mikroorganismen der Gattung Rhizobium erhältlich ist.

3. Restriktionsendonuklease nach Anspruch 1 oder 2 dadurch gekennzeichnet, daß sie aus Rhizobium leguminosarum DSM 5629 erhältlich ist.

4. Restriktionsendonuklease nach den Ansprüchen 1 bis 3, gekennzeichnet durch ein Temperatur-Optimum bei 30°C und ein pH-Optimum zwischen pH 7,5 und 8,0.

5. Verfahren zur Gewinnung einer TypII-Restriktionsendonuklease mit der Erkennungssequenz und der durch die Markierung charakterisierten Spaltstelle

$$5'\text{-CCC ACA NNNNNNNN NNN} | \text{N-3'}$$
$$3'\text{-GGG TGT NNNNNNNN} | \text{NNN N-5'}$$

(N:A,G,C oder T)

dadurch gekennzeichnet, daß man Mikroorganismen der Gattung Phizobium züchtet und das Enzym aus den Zellen gewinnt.

6. Verfahren nach Anspruch 5, dadurch gekennzeichnet, daß man Phizobium leguminosarum DSM 5629 züchtet.

7. Verfahren nach den Ansprüchen 5 und 6, dadurch gekennzeichnet, daß man die Zellen aufschließt und den Zellüberstand gewinnt.

8. Verfahren nach Anspruch 7, dadurch gekennzeichnet, daß man zur Feinreinigung den Zellüberstand einer Affinitätschromatographie und einer Anionenaustauschchromatographie unterwirft.

9. Verfahren nach Anspruch 8, dadurch gekennzeichnet, daß man zur Affinitätschromatographie trägerfixiertes Heparin verwendet.

10. Verwendung einer TypII-Restriktionsendonuklease mit der Erkennungssequenz (SEQ ID NO 1) und der durch die Markierung charakterisierten Schnittstelle

$$5'\text{-CCC ACA NNNNNNNN NNN} | \text{N-3'}$$
$$3'\text{-GGG TGT NNNNNNNN} | \text{NNN N-5'}$$

(N:A,G,C oder T)

zur Erkennung und Spaltung der doppelsträngigen DNA-Sequenz $5'\text{-CCCACA(N)}_{12/9}\text{-}3'$, oder der komplementären Sequenz.

SEQ ID NO 1

ART DER SEQUENZ: Nukleotid
SEQUENZLÄNGE: 18 Basenpaare

STRANGFORM: Doppelstrang
TOPOLOGIE: linear

MERKMALE:
von 7 bis 18 N: A, G, C oder T


CCCACANNNN NNNNNNNN                          18

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | GENE. vol. 95, no. 1, 30 Oktober 1990, AMSTERDAM NL Seiten 129 - 131; VESELY, Z. et al.: "RleAI: a novel class-IIS restriction endonuclease from Rhizobium leguminosarum recognizing 5'-CCCACA(N)12-3' 3'-GGGTGT(N)9 -5'" * das ganze Dokument * | 1-10 | C12N9/22 C12N1/20 //(C12N1/20, (C12R1:41) |

**RECHERCHIERTE SACHGEBIETE (Int. Cl.5 )**

C12N

Der vorliegende Recherchenbericht wurde für alle Patentansprüche erstellt

| Recherchenort | Abschlußdatum der Recherche | Prüfer |
|---|---|---|
| DEN HAAG | 12 FEBRUAR 1991 | ANDRES S.M. |

EPO FORM 1503 03.82 (P0403)